(19) Europäisches Patentamt
European Patent Office
Office européen des brevets

(11) EP 3 056 195 A1

(12) EUROPEAN PATENT APPLICATION

(43) Date of publication:
**17.08.2016 Bulletin 2016/33**

(21) Application number: **15154971.4**

(22) Date of filing: **13.02.2015**

(51) Int Cl.:
***A61K 9/00*** *(2006.01)* ***A61Q 11/00*** *(2006.01)*
***A61K 8/73*** *(2006.01)* ***A61K 31/716*** *(2006.01)*
***A61K 31/719*** *(2006.01)* ***A61K 31/728*** *(2006.01)*
***A61K 9/06*** *(2006.01)* ***A61K 8/04*** *(2006.01)*
*A61K 31/723 (2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Vision:Organic GmbH**
**32052 Herford (DE)**

(72) Inventors:
- **SCHÖTTELNDREIER, Dirk**
  **33615 Bielefeld (DE)**
- **UTHMANN, Stefan**
  **33790 Halle (DE)**

(74) Representative: **Vos, Derk**
**Maiwald Patentanwalts GmbH**
**Elisenhof**
**Elisenstraße 3**
**80335 München (DE)**

# (54) COMPOSITIONS COMPRISING HYALURONIC ACID AND BETA-GLUCAN FOR TOPICAL APPLICATIONS IN ORAL CAVITY

(57) The invention relates to compositions comprising hyaluronic acid, beta-glucan and optionally pullulan. The invention relates to the use of the compositions as cosmetic compositions for use in oral hygiene. Further, the invention relates to the compositions as pharmaceutical compositions for use in the topical application in the treatment or prophylaxis of inflammatory affections, irritations, hypersensitivities or viral infections of the oral cavity.

EP 3 056 195 A1

## Description

### FIELD OF INVENTION

The present invention relates to compositions for topical applications in the oral cavity to treat or prevent inflammatory affections including aphthae.

### BACKGROUND OF THE INVENTION

Inflammatory affections, hypersensitivities or irritations of the mouth may occur under various circumstances.

Inflammation of the mucous lining of any of the structures of the mouth may involve the cheeks, gums, tongue, lips, and roof or floor of the mouth. The inflammation, also often referred to as stomatitis, can be induced by conditions of the mouth itself, such as poor oral hygiene, poorly fitted dentures, or from mouth bums due to hot food or beverages, allergic reactions, or infections. The inflammation is usually associated with redness, swelling, and occasional bleeding from the affected area.

Poorly fitted oral appliances, cheek biting, or jagged teeth can also persistently irritate the oral structures. Further, chronic mouth breathing due to plugged nasal airways can cause dryness of the mouth tissues, which in turn leads to irritation. Drinking beverages that are too hot can burn the mouth, leading to irritation and pain. In addition, diseases, such as herpetic infections (the common cold sore) or systemic diseases associated with stomatitis include inflammatory bowel disease (IBD) and or an inflammatory multisystem disorder of unknown cause, may also result in an inflammation of the oral cavity. Stomatitis can also be caused or intensified by pharmacological, particularly chemotherapeutic treatments and by radiotherapy.

Stomatitis can range from mild to severe and patients with severe stomatitis are unable to take anything by mouth.

Further, aphthous stomatitis, also known as recurrent aphthous ulcers (RAU) or canker sores, is a specific type of stomatitis that is characterized by shallow, painful ulcers that are usually located on the lips, cheeks, gums, or roof or floor of the mouth. These ulcers can range from pinpoint size to up to 1 inch (2.5 cm) or more in diameter.

Parodontitis, also called Periodontitis, is a set of inflammatory diseases affecting the periodontium, i.e., the tissues that surround and support the teeth. Periodontitis involves progressive loss of the alveolar bone around the teeth, and if left untreated, can lead to the loosening and subsequent loss of teeth. Periodontitis is caused by microorganisms that adhere to and grow on the tooth's surfaces, along with an overly aggressive immune response against these microorganisms. Periodontitis can be divided into the subclasses of gingivitis, chronic periodontitis, aggressive periodontitis, periodontitis as a manifestation of systemic disease, necrotizing ulcerative gingivitis/periodontitis, abscesses of the periodontium and combined periodontic-endodontic lesions.

The primary cause of gingivitis is poor or ineffective oral hygiene which may lead to the accumulation of a mycotic and bacterial matrix at the gum line, called dental plaque. Other contributors are poor nutrition and underlying medical issues such as diabetes. Diabetics must be meticulous with their homecare to control periodontal disease. Another risk factor is smoking.

Furthermore, the tissue of the oral cavity may also be damaged due to traumatizing events such as teeth removal surgery, professional tooth cleaning (PTC) or other surgical procedures within the oral cavity.

In general, a common method to prevent inflammatory affections of the oral cavity is oral hygiene, including regular brushing of the teeth, also using interdental brushes or antiseptic mouthwashes.

The restoration and healing of the damaged tissue due to an inflammation of the gingiva and/or the mucosa may be accelerated by the topical application of compositions containing a film former, wherein the affected area is physically covered by a film that protects from further stresses interfering with the healing process.

Further, these compositions may also comprise hyaluronic acid which may support the healing process of the affected tissue (e.g. see EP 0 444 492 A1). For example, hyaluronic acid may be found in Gengigel® products from Ricerfarma.

Typically, the compositions further comprise bactericidal components to accelerate the healing process.

Furthermore, WO2005/00321 A1 describes compositions for the treatment of oral cavity aphthae, wherein hyaluronic acid is the sole active ingredient.

However, there is still a need for compositions in the treatment of the oral cavity that are more effective and/or more consumer-friendly (i.e. compositions with less side effects, e.g. inducing less irritations after topic application or providing a more comfortable feeling inside the oral cavity or a more comfortable smell or taste of the composition) than the already existing commercially available compositions.

It is thus an object of the present invention to provide a composition for topical application in the treatment or prevention of inflammation of the oral cavity.

It is another object of the present invention to provide a composition comprising film forming agents of natural origin, i.e. that were not chemically synthesized or chemically modified but derived from natural products or which were

produced biotechnologically.

It is another object of the present invention to provide improved compositions for topical applications in the treatment or prophylaxis of inflammation of the oral cavity.

## SUMMARY

The objectives of the present invention are solved by a composition comprising hyaluronic acid or a salt thereof, beta-glucan and optionally pullulan.

The compositions may be pharmaceutical or cosmetic compositions.

Further, the invention relates to the use of compositions in the acute or prophylactic treatment of inflammatory affections, hypersensitivity, irritations or viral infections of the oral cavity. The treatment is oftentimes indicated after traumatizing events or physical stress such as professional teeth cleaning, dental surgery, chemotherapy, radiotherapy or it becomes necessary due to poor oral hygiene.

The invention also relates to the use of hyaluronic acid with a molecular weight ranging from about 50 kDa to about 3,000 kDa or a salt thereof in the preparation of a pharmaceutical composition for topical application in the acute or prophylactic treatment of inflammatory affections, hypersensitivity, irritations or viral infections of the oral cavity.

## FIGURE LEGEND

Figure 1: Size exclusion chromatography coupled with multi angle light scattering (SEC-MALS) chromatogram of 0.5 % w/v Cube3® in 50mM NaPi, 100mM NaCl, pH 7.45.

## DETAILED DESCRIPTION

The present invention relates to a composition comprising

(i) hyaluronic acid or a salt thereof,
(ii) beta-glucan, and
(iii) optionally pullulan.

Preferably, hyaluronic acid, a salt thereof, beta-glucan and pullulan are the active ingredients of the composition. Preferably, they may function as film forming agents. In a preferred embodiment, the hyaluronic acid or salt thereof has a molecular weight ranging from about 10 kDa to about 5,000 kDa and preferably from about 50 kDa to about 3,500 kDa. Preferably, the hyaluronic acid or salt thereof is sodium hyaluronate. In a particularly preferred embodiment, the hyaluronic acid is CUBE3® by Principium S.A.

In a particularly preferred embodiment, the hyaluronic acid or salt thereof has a weight average molar mass ($M_W$) from about 50 kDa to about 750 kDa, more preferably from about 300 kDa to about 725 kDa and even more preferably from about 500 kDa to about 700 kDa. The $M_W$ may be determined by SEC-MALS.

In a preferred embodiment, the composition of the present invention comprises from about 0.01 wt% to about 5 wt% hyaluronic acid or salt thereof. Preferably, the composition comprises from about 0.1 wt% to about 1 wt% hyaluronic acid or salt thereof.

In another preferred embodiment, the composition comprises beta-glucan with an average molecular weight from about 250 kDa to about 1,500 kDa. Preferably, the average molecular weight of the beta-glucan is from about 250 kDa to about 350 kDa.

The beta-glucan of the composition according to the present invention is preferably unmodified. Typically, beta-glucan may be produced from fungi, yeast or oat. However, due to a branched structure, beta-glucan often has a low solubility in water. The solubility may then be improved by chemical modifications such as sulfonation with $H_2SO_4$. However, the chemical modification may change the molecular structure of the glucan and may also affect its efficacy. A preferred chemically unmodified beta glucan is, for example, the yeast-based PUROLAN® by Lanxess or the oat-based SymGlucan® of Symrise AG. Preferably, the composition according to the present invention comprises from about 0.1 wt% to about 20 wt% (wt% are based on the overall weight of the composition) of a 5 wt% aqueous beta-glucan solution (wt% are based on total weight of aqueous beta-glucan solution). Preferably, the composition according to the present invention comprises from about 0.2 wt% to about 10 wt% of a 5 wt% aqueous beta glucan solution.

In yet another preferred embodiment, the composition of the present invention comprises from about 0.1 wt% to about 20 wt% pullulan. Preferably, the composition comprises 0.1 wt% to about 10 wt% pullulan and more preferably, from about 0.5 wt% to about 5 wt% pullulan. A preferred pullulan is, for example, Pullulan USP-NF of Hayashibara Ltd.

In a particularly preferred embodiment, the composition according to the present invention comprises from about 0.2 wt% to about 10 wt% beta-glucan (5 wt% aqueous solution), from about 0.1 wt% to about 1 wt% sodium

hyaluronate and from about 0.1 wt% to about 1 wt% pullulan, wherein the wt% of beta-glucan, sodium hyaluronate and pullulan are based on the total weight of the composition of the present invention.

In another embodiment, the composition according to the present invention comprises at least one further naturally occurring film forming agent such as Guar gum, Carageenan or Acacia Senegal gum.

In an alternative embodiment, the composition according to the present invention is applied as a patch, wherein the patch comprises the composition according to the present invention and a dissolvable polymer. The polymer may dissolve under conditions of the oral cavity (for example temperature of about 37 °C, pH 6-7, contacting saliva etc.). Preferably, the polymer is selected from the group consisting of and starch acetate, cellulose derivatives, such as hydroxypropylcellulose, ethylcellulose, sodium carboxmethylcellulose; and combinations thereof. Preferably, the polymer dissolves or substantially dissolves (i.e. preferably more than 90 % of the polymer has dissolved or alternatively, the polymer dissolved so that the patient does not "feel" the patch any more) within the oral cavity within 15 minutes, within 30 minutes or within 60 minutes after topical application of the composition. Preferred examples of dissolvable polymers for patches are Rapidfilm® polymers of Tesa Labtec GmbH.

In another particularly preferred embodiment, the composition according to the present invention does not comprise chemically synthesized film forming agents. Examples of chemically synthesized film forming agents are polyvinylpyrrolidone (PVP) and polyvinylalcohol (PVA). In one aspect of the present invention, the active film forming agents are derived from natural sources or are produced by biotechnological methods.

In another embodiment, the composition according to the present invention does also not comprise ethanol, polyethylene glycol, halogenated preservatives, parabens, synthetic dyes or synthetic sweeteners. Examples of halogenated preservatives are chlorohexidines, triclosanes, dichlorocarbanes, climbazoles, 2-Bromo-2-nitropropane-1,3-diols, dichlorobenzyl alcohols, methylchloroisothiazolinones, chlorphenesin and benzalkonium chlorides.Examples of synthetic dyes are brilliant blue, Tartrazine, Quinoline Yellow, Sunset Yellow, Amaranth, Ponceau 4R. Examples of parabens are methylparaben (E number E218), ethylparaben (E214), propylparaben (E216), butylparaben, isopropylparaben, phenylparaben and isobutylparaben. Examples of synthetic sweeteners are aspartame, sucralose, neotame, acesulfame potassium, and saccharin. It is known that the use of Aspartame is controversial and studies have shown that it has carcinogenic potential. Sucralose is a chloro-organic compound and Saccharin has been shown to induce cancer in animal studies. Saccharin and Acesulfam have a bitter aftertaste.

In another preferred embodiment, the composition according to the present invention is a pharmaceutical composition. The composition may then provide a protective film covering areas after topical application in the oral cavity. The composition may be used in the acute and/or prophylactic treatment. The composition may be used in the preparation of a medicament for the treatment of viral Herpes infections that occur at the oral cavity, and particularly at the lips, such as Herpes infections causing Herpes labialis. The composition according to the present invention may then be applied topically intra and extraoral of the lips. Further, the pharmaceutical composition according to the present invention may also be used in the preparation of a medicament in the treatment of inflammatory affections occurring in the oral cavity.

Such inflammatory affections may be present in the form of a gingivitis, stomatitis, aphthous diseases, periimplantitis, mucositis, periodontitis. Furthermore, inflammatory affections or irritations or hypersensitivities to be treated may also have been induced by chemotherapy, radiotherapy or traumatizing events such as dental surgery or professional tooth cleaning.

In another preferred embodiment, the composition according to the present invention is used as a cosmetic composition, preferably as a cosmetic composition for the oral cavity. Preferably, the composition is applied to maintain oral hygiene, such as dental care, avoiding bad breath or dental decay.

In yet another preferred embodiment, the pharmaceutical composition may be applied topically in the form of a gel, a spray solution, a paste, an adhesive paste, a powder or mouthwash. In an alternative embodiment the pharmaceutical composition is applied as a patch.

Furthermore, the present invention also relates to the use of hyaluronic acid with a molecular weight ranging from about 50 kDa to about 3,500 kDa or a salt thereof in the preparation of a pharmaceutical composition for topical application in the acute or prophylactic treatment of inflammatory affections, hypersensitivity, irritations or viral infections of the oral cavity. In a preferred embodiment, the $M_W$ of the hyaluronic acid is from about 50 kDa to about 750 kDa, preferably from about 300 kDa to about 725 kDa and even more preferably from about 500 kDa to about 700 kDa.

As used herein, the term "hyaluronic acid" refers to a non-sulfated glucosaminoglycan which is a polymer of disaccharides of D-glucuronic acid and D-N-acetylglucosamine, linked via alternating β-(1,4) and β-(1,3) glycosidic bonds. The structure of hyaluronic acid is shown below in Formula (I).

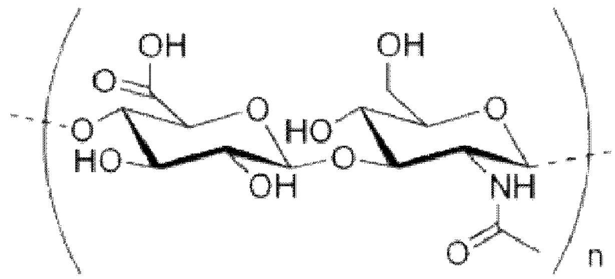

Formula (I)

Polymers of hyaluronic acid can range in size from about 5 kDa to about 20,000 kDa. The term hyaluronic acid as used herein refers to all polymers of hyaluronic acids in their different sizes and mixtures thereof. Typically, it is not feasible to characterize hyaluronic acid by a specific molecular weight since it is normally present as a mixture of polymers of different lengths. Consequently, hyaluronic acid as used herein may either be characterized by the average molecular weight of hyaluronic acid (e.g. average molecular of 10 kDa for hyaluronic acid of equal amounts of polymers ranging from 7 kDa to 13 kDa) and/or by the range of the molecular weight of hyaluronic acid (e.g. ranging from 5 kDa to 5,000 kDa).

As used herein, the term "beta-glucan" refers to polysaccharides of D-glucose monomers linked by any kind of β-glycosidic bonds. Some β-glucan compounds are continual repeats of D-glucose attached at a specific position, while other beta-glucans may be branched and comprise different kinds of β-glycosidic bonds. Common examples of β-glycosidic bonds are β(1-3), β(1-4) or β(1-6) glycosidic bonds.

The designation of β(1-3) for a glycosidic linkage indicates that the etheric oxygen bridge between two consecutive monosaccharide units of the polysaccharide connects the number 1 carbon of the first unit to the number 3 carbon of the second unit, and that etheric oxygen bridge attaches to carbon 1 of the first unit from above the ring.

The designation of β(1-4) for a glycosidic linkage indicates that the etheric oxygen bridge between two consecutive monosaccharide units of the polysaccharide connects the number 1 carbon of the first unit to the number 4 carbon of the second unit, and that etheric oxygen bridge attaches to carbon 1 of the first unit from above the ring.

Likewise, the designation of β(1-6) for a glycosidic linkage indicates that the etheric oxygen bridge between two consecutive monosaccharide units of the polysaccharide connects the number 1 carbon of the first unit to the number 6 carbon of the second unit, and that etheric oxygen bridge attaches to carbon 1 of the first unit from above the ring.

Non-limiting examples of beta-glucans include cellulose and other D-glucose polysaccharides that can, for example, be extracted from the bran of cereal grains (e.g. oat or barley), the cell wall of baker's yeast, certain fungi, mushrooms and bacteria.

It is noted that the structure of beta-glucans depends on the organism out of which the beta-glucan is extracted. For example, oat is a rich source of the water-soluble fibre (1,3/1,4) β-glucan. However, beta-glucan extracted from baker's yeast is typically rich in (1,3/1,6) β-glucan. In the context of the present invention, the term beta-glucan comprises all possible polymer structures comprising any kind of glycosidic bond, comprising branched and unbranched β-D-glucose polysaccharides.

As used herein, the term "pullulan" refers to a polysaccharide polymer consisting of maltotriose units wherein three glucose units of the maltotriose are connected by an α-(1,4) glycosidic bond and consecutive maltotriose units are connected to each other by an α-(1,6) glycosidic bond. The basic structure of a maltotriose unit of pullulan is shown below as Formula (II).

Formula (II)

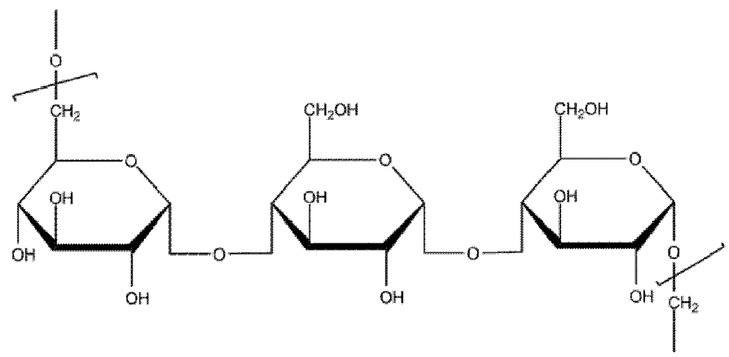

Pullulan occurs naturally as a cell wall component of the fungus *Aureobasidium pullulans.* It may be produced from starch or sugar in a fermentation process by *Aureobasidium pullulans*. As a food additive, it is known by the E number E1204.

As used herein, the term "salt" refers to salt formed from an acid with a basic group. Salts are composed of related numbers of cations (positively charged ions) and anions (negative ions). Preferably, hyaluronic acid salts refer to salts wherein hyaluronic acid is the anion. Non-limiting examples of hyaluronic acid salts are sodium hyaluronate and tetra-n-butyl ammonium hyaluronate.

As used herein, the term "oral cavity" refers to the first portion of the digestive tract, comprising the mouth and the structures which are enclosed, including but not limited to, the lips, dentin, gingiva, roof of the mouth, tongue, inside of cheeks and the buccal cavity of an animal or a human.

The term "parabens" as used herein refers to esters of para-hydroxybenzoic acid of formula (III),

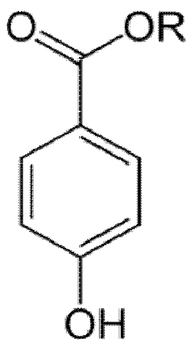

,

Formula (III)

wherein R is an alkyl-group such as ($C_1$-$C_6$)-alkyl group. It is known that in individuals with normal skin, parabens are, for the most part, non-irritating and non-sensitizing. However, parabens may cause skin irritation and contact dermatitis and rosacea in individuals with paraben allergies, which occur in a certain percentage of the general population. Consequently, parabens are still subject of ongoing toxicology studies.

The term "professional tooth cleaning" (PTC) as used herein refers to a procedure for the removal of tartar (mineralized plaque) that may develop even with careful brushing and flossing, especially in areas that are difficult to reach in routine tooth brushing. It is often done by a dental hygienist. Professional tooth cleaning includes tooth scaling and tooth polishing and debridement if too much tartar has accumulated. This involves the use of various instruments or devices to loosen and remove deposits from the teeth. However, it is known that professional tooth cleaning always comes along with mechanical thermal and/or osmotic stress inside the oral cavity and thus may lead to (temporary) hypersensitivity and irritations of the teeth and gingiva, and (temporarily) increases the risk of an inflammatory infections of the oral cavity.

As used herein, the term "mouthwash" refers to a solution used as an effective home care system to enhance oral hygiene.

The term "oral hygiene" refers to the practice of keeping the mouth and teeth clean to prevent dental problems, such as dental cavities, gingivitis, and bad breath. There are also oral pathologic conditions in which good oral hygiene is required for healing and regeneration of the oral tissues. These conditions include gingivitis, periodontitis and dental trauma, such as subluxation, oral cysts, and following wisdom tooth extraction. Methods of oral hygiene may include, but are not limited to, cleaning of teeth, cleaning of the tongue, gum care and oral irrigation.

As used herein, the term "preservative" as in "halogenated preservative" or "bactericide" as in "halogenated bactericides" refers to a naturally occurring or synthetically produced substance that is added to products such as foods, pharmaceuticals, paints, biological samples, wood, etc. to prevent decomposition by microbial growth or by undesirable chemical changes. Non-limiting examples are chlorohexidines, triclosanes, dichlorocarbanes, climbazoles, 2-Bromo-2-nitropropane-1,3-diols, dichlorobenzyl alcohols, methylchloroisothiazolinones, chlorphenesin and benzalkonium chlorides.

As used herein, the term "gel" refers to a non-fluid colloidal network or polymer network that is expanded throughout its whole volume by a fluid. As used herein, the fluid trapped inside the gel may comprise the composition according to the present invention.. The network structure may result from physical bonds (physical gels) or chemical bonds (chemical gels), as well as crystallites or other junctions that remain intact within the extending fluid. The network structure may also comprise components of the composition of the present invention.

The term "paste", as used herein, refers to a thick viscous fluid. As used herein, the paste comprises the

composition according to the present invention. A paste may be realized by standard methods of mixing the composition according to the present invention with further additives known to the skilled person.

The term "adhesive powder" refers to a material composed of very fine particles that are not cemented together and which are adhesive when applied to the oral cavity.

As used herein, the term "spraying solution" refers to a composition which is capable of being dispersed as a fine jet of small drops of or liquid vapor discharged from a pressurized container.

As used herein, the term "film forming agent" refers to a compound or composition capable of building a thin layer covering the area of application, for example within the oral cavity. Film forming agents can either be chemically synthesized film forming agents or naturally occurring film-building agents or biotechnological film forming agents. Examples of chemically synthesized film forming agents are polyvinylpyrrolidone, polyvinylalcohol acrylate copolymers, acrylate crosspolymer, acrylamides copolymers or acetyl triethyl citrate. Preferably, the film forming agents of the present invention are naturally occurring or biotechnologically produced film-building agents. Examples of preferred naturally occuring or biotechnologically produced film forming agents are hyaluronic acid, Glucans, pullulan, acacia Senegal gum, acacia Catechu gum or acacia Farnesiana gum.

As used herein, the term "naturally occurring" refers to compounds that may be extracted from natural sources such as plants, bacteria, yeast, soil or animals. Preferably, the naturally occurring compounds comply with the requirements of the leading natural cosmetic or organic cosmetic certifications "COSMOS" (Version 2.0 as of October 21, 2013) of COSMOS-standard AISBL and "NATRUE" (Version 2.8 as of June 5, 2013) of the International Natural and Organic Cosmetics Association A.I.S.B.L . Typically, the natural source is easily obtainable in large quantity and allows the extraction of the compounds in high amounts suitable for mass production and not only in small quantities suitable for analytical purposes. Examples of naturally occurring compounds include, but are not limited to, hyaluronic acid, cellulose, beta glucan, pullulan and xylans.

As used herein, the term "biotechnologically produced" or "produced by biotechnological methods" refers to compounds that are produced on an industrial scale by living organisms. Non-limiting examples of biotechnologically produced compounds are pullulan produced in a fermentation process by *Aureobasidium pullulans,* xylan produced in a fermentation process comprising microbial xylanase, recombinant proteins produced in bacteria such as *E.coli* and genetically modified crops. Further examples are lactic acid produced by *lactobacillus* and hyaluronic acid produced by *streptococcus zooepidemicus*.

As used herein, the term "chemically synthesized" refers to compounds obtained by a purposeful execution of chemical reactions to obtain a product, or several products, wherein the synthesis does not exclusively involve biotechnological methods or merely the extraction of naturally occurring sources as described above. The term "chemically synthesized" also includes compounds that were produced by processes that involve chemical reaction steps as well as reaction steps involving the use of biomolecules that have been biotechnologically produced (e.g. the use of enzymes as catalysts in the synthesis of compounds that also include chemical reaction steps such as halogenation or nitrosylation of the compound).

As used herein, the term "traumatizing event" or "traumatizing events" refers to the occurrence of an unusual event that affects the oral cavity, for example due to the application of force, pressure, osmotic stress, thermal or chemical agents. Non-limiting examples of traumatizing events include, but are not limited to, professional teeth cleaning, dental surgery, other surgical procedures within the oral cavity, fracture of the jaw, loss of teeth, drinking of very hot or very cold beverages, drinking of a strong acid, base, or drinking of other chemical agents such as preservatives which are not intended for oral intake, sharp objects such as sharp crumbs in oral cavity, or bite wounds.

As used herein, the term "irritation" or "irritations" refers to a response of a cell, nerve, human tissue, or muscle to a stimulus which may result in an inflammatory affection.

As used herein, the term "inflammatory affection" refers to a response and/or a reaction of the immune system to an irritation. The reaction may take place in the affected body organ, in surrounding connective tissues, the affected blood vessels and/or the adjacent lymphatic system. Typical signs for an inflammatory affection are rubor, calor, tumor, dolor and a following functio laesa.

As used herein, the term "hypersensitivity" refers to a condition, wherein at least part of the oral cavity reacts abnormally strong to external stimuli. A non-limiting example of hypersensitivity is dentin hypersensitivity, which is dental pain which is sharp in character and of short duration, arising from exposed dentin surfaces in response to stimuli, typically thermal, evaporative, tactile, osmotic, chemical or electrical and which cannot be ascribed to any other dental disease. Typical triggers of dentin hypersensitivity are hot and cold drinks and foods, acids or bases, cold air or a coolant water jet from a dental instrument.

As used herein, the term "osmotic stimulus" or "osmotic stimuli" refers to a stimulus that is based on osmosis, wherein the differences in osmolarity and the subsequent flow of water may be stimulated.

As used herein, the term "viral infection" or "viral infections" refer to the infection of the host by a virus wherein the infection affects the oral cavity. A non-limiting example of a viral infection is Herpes labialis which is a type of herpes simplex infection occurring on the lip. An outbreak typically causes small blisters or sores on or around the mouth. The

sores typically heal within 2-3 weeks, but the herpes virus remains dormant in the facial nerves and periodically the virus reactivates and creates new sores in the same area of the mouth or face at the site of the original infection.

The present invention also relates to the following items:

(1) A composition comprising

(i) hyaluronic acid or a salt thereof,
(ii) beta-glucan, and
(iii) optionally pullulan.

(2) The composition of item (1), wherein hyaluronic acid or salt thereof has a molecular weight ranging from about 50 kDa to about 3,500 kDa.
(3) The composition of item (1) or (2), wherein the hyaluronic acid or salt thereof has a weight average molar mass from about 50 kDa to about 750 kDa.
(4) The composition of any one of items (1) to (3), wherein the composition comprises from about 0.1 wt% to about 1 wt% hyaluronic acid or salt thereof.
(5) The composition of any one of items (1) to (4), wherein the hyaluronic acid or salt thereof is sodium hyaluronate.
(6) The composition of items (1) to (5), wherein beta-glucan has an average molecular weight from about 250 kDa to about 1250 kDa.
(7) The composition of any one of items (1) to (6), wherein the beta-glucan is chemically unmodified.
(8) The composition of any one of items (1) to (7), wherein the composition comprises from about 0.1 wt% to about 20 wt% beta-glucan.
(9) The composition of any one of items (1) to (8), wherein the composition comprises from about 0.1 wt% to about 10 wt% pullulan.
(10) The composition of any one of items (1) to (9), wherein the composition further comprises another naturally occurring film forming polymer.
(11) The composition of item (1) to (10), wherein the composition does not comprise a chemically synthesized film forming agent selected from the group consisting of polyvinylpyrrolidone (PVP) acrylate copolymers, acrylate crosspolymers, acrylamide coplymers, acetyl triethyl citrate and polyvinylalcohol (PVA).
(12) A composition of any one of item (1) to (11), wherein the composition does not comprise chemically synthesized film forming agents.
(13) The composition of any one of items (1) to (12), wherein the composition does not comprise ethanol, polyethylene glycol, parabens, halogenated preservatives, synthetic dyes and/or synthetic sweeteners.
(14) A composition of any one of items (1) to (13) in the form of a gel, a paste, a spray solution, mouthwash, adhesive paste or powder.
(15) A patch comprising the composition of any one of items (1) to (14), wherein the patch further comprises a dissolvable polymer.
(16) The patch of item 15, wherein the polymer dissolves inside the oral cavity.
(17) The patch of item (16), wherein the polymer is dissolved or substantially dissolved within 15 minutes, within 30 minutes or within 60 minutes after topical application in the oral cavity.
(18) The patch of item (15) to (17), wherein the polymer is selected from the group consisting of cellulose derivatives, starch acetate and combinations thereof.
(19) A composition of any one of items (1) to (14), wherein the composition is a cosmetic composition.
(20) Use of a composition of any one of items (1) to (14) and (19) for oral hygiene.
(21) A composition of any one of items (1) to (14), wherein the composition is a pharmaceutical composition.
(22) Use of a composition of any one of items (1) to (14) in the preparation of a pharmaceutical composition for topical application in the acute or prophylactic treatment of inflammatory affections, hypersensitivity, irritations or viral infections of the oral cavity.
(23) Use of a composition of item (22) in the preparation of a pharmaceutical composition of item (22), wherein the inflammatory affection is selected from the group consisting of peridontitis, gingivitis, stomatitis and aphthous stomatitis, periimplantitis and mucositis.
(24) Use of a composition of item (22) in the preparation of a pharmaceutical composition of item (22), wherein the acute or prophylactic treatment occurs after chemotherapy, radiotherapy or traumatizing events.
(25) Use of a composition of item (24) in the preparation of a pharmaceutical composition of item (24), wherein the acute or prophylactic treatment occurs after professional tooth cleaning, dental surgery, chemotherapy or radiotherapy.
(26) Use of a composition of item (22) in the preparation of a pharmaceutical composition of item (22), wherein the prophylactic or acute treatment is the treatment of Herpes infections, preferably the treatment of Herpes labialis.

(27) Composition of any one of items (1) to (14) for use as a medicament.

(28) Composition of any one of items (1) to (14) and (27) for use in the acute or prophylactic treatment of inflammatory affections, hypersensitivity, irritations or viral infections of the oral cavity.

(29) Composition for use of item (28), wherein the inflammatory affection is selected from the group consisting of peridontitis, gingivitis, stomatitis, aphthous stomatitis, periimplantitis and mucositis.

(30) Composition for use of item (29), wherein the acute or prophylactic treatment occurs after chemotherapy, radiotherapy or traumatizing events.

(31) Composition for use of item (30), wherein the acute or prophylactic treatment occurs after professional tooth cleaning, dental surgery, chemotherapy or radiotherapy.

(32) Composition for use of item (29), wherein the prophylactic or acute treatment is the treatment of Herpes infections, preferably the treatment of Herpes labialis.

(33) Use of hyaluronic acid with a molecular weight ranging from about 50 kDa to about 3,500 kDa or a salt thereof in the preparation of a pharmaceutical composition for topical application in the acute or prophylactic treatment of inflammatory affections, hypersensitivity, irritations or viral infections of the oral cavity and wherein preferably the hyaluronic acid or salt thereof has a weight average molar mass from about 50 kDa to about 750 kDa.

EXAMPLES

### Example 1: formulation

Formulation 1: water (about 75 wt%), glycerol (about 15 wt%), propyleneglycol (Zemea ® from DuPont) (about 5 wt%), xanthan gum (about 1 wt%), pullulan (about 1 wt%), (1,3)(1,6)-β-glucan (about 2 wt% of 5 wt% Purolan® aqueous solution), sodium hyaluronate Cube3® (about 0.3 wt%), potassium sorbate (< 1 wt%), polyglyceryl-4 caprate (< 1 wt%), sodium lactate (< 1 wt%), stevia rebaudiana (about 0.05 wt %), aroma (about 1 wt%), 2-Phenylethylalkohol (< 1 wt%) and parabens (0.15 wt%; Nipaset, Clariant).

Phase A of the gel of Formulation 1 is prepared by stirring up to complete dispersion the Xanthan gum, glycerin and 1,3 propanediol (Zemea) in about 70 % of the total water at room temperature.

Phase B is prepared by adding sodium hyaluronate and pullulan to 20 % of total water and stirring till complete dissolution. Then phase B is added to phase A followed by adding Purolan (water, (1,3)(1,6)-β-glucan and 2-Phenylethyl alcohol) to phase A and B. Phase C is prepared by adding Stevia rebaudiana to 10 % of total water and stirring till total dissolution before adding to phase A and B.

Polysol KP Plus (aqua, potassium sorbate, glycerin, polyglyceryl-4 caprate, sodium lactate) and the aroma are added to phase A, B and C under stirring. The paraben is subsequently added to the formulation.

Reference 1: Aqua, Xylitol, Carboxymethylcellulose, alcohol, PEG-40 hydrogenated castor oil, polyvinylalcohol, (2,4-dichlorphenyl)methanol, aroma, sodium hyaluronate (0.2 wt%), brilliant blue, polycarbophil, sodium hydroxide. Reference 1 is commercially available as Gengigel® by Ricerfarma.

Placebo: similar to Formulation 1 but without sodium hyaluronate, (1,3)(1,6)-β-glucan, and pullulan. The amount of xanthan gum is increased respectively.

### Example 2: study setup

26 subjects were randomly divided into three groups. First, the Sulkulus bleeding index (SBI), and pocket depth (6-point measurement) were determined for each patient. The person skilled in the art knows how to perform the pocket depth measurement. The SBI was determined as the modified SBI according to Mombelli, A et al; Oral microbial Immunol. 2, 145-151; 1987. Next, a professional tooth cleaning (PTC) was performed for each subject. Each group was then treated with a different gel, namely a placebo (n=6), reference 1 (n=10) and formulation 1 (n=10), in a double-blinded study. All products were provided in neutral packaging.

The gel was homogenously applied on the teeth and the gingiva (on the lingual as well as on the vestibular side of the teeth/gingiva) of each subject using a cotton swab. The gel was applied by the patient after being instructed by a healthcare professional.

The gel was applied:

a. directly after PTC;
b. in the evening on the same day;
c. twice daily in the morning and in the evening until 7 days (d7) after PTC;
d. the morning of d8 after PTC.

**Example 3: topical application after professional tooth cleaning (after 24h)**

Patients were interviewed by telephone 24h after PTC. They were asked the following 6 questions, each of them was to be answered by selecting out of 5 options, wherein option 1 was always the most favorable and 5 the least favorable answer.

I) How does the gel feel like after the first application inside the mouth?

II) How do you like the taste and the smell of the gel after the first application inside the mouth?

III) How do you like the appearance of the gel?
Options for answering questions I) to III): 1=very comfortable, 2=comfortable, 3=neutral, 4=uncomfortable,5=very uncomfortable.

IV) In case you experienced irritations after the tooth cleaning, did they go away after the first three applications of the gel?
1=completely, 2=almost completely, 3=partly, 4=rather only a little, 5=rather not at all.

V) Would you like to use the gel again after the next tooth cleaning?
1=definitely, 2=almost definitely, 3=undecided, 4=rather not, 5=by no means.

VI) If you had tooth cleanings or dental clearances in the past, was the overall progress applying the gel...
1=very much better, 2=better, 3=the same, 4=worse , 5=very much worse than without applying the gel.

The results of the telephone interviews are shown below in Table 1.

**Table 1: Overview of average score of questionnaire 1 day after PTC.**

| | Average score | | |
|---|---|---|---|
| **Question** | **Placebo** | **Reference 1** | **Formulation 1** |
| **I** | 2.8 | 2.6 | 2.5 |
| **II** | 2.7 | 3.2 | 2.3 |
| **III** | 2.5 | 3.1 | 2.6 |
| **IV** | 2.2 | 3.1 | 2.3 |
| **V** | 2.7 | 3.1 | 2.4 |
| **VI** | 3.2 | 3.1 | 2.1 |

**Example 4 topical application after professional tooth cleaning (after 7d)**

Patients were interviewed at the practice 7 days after PTC. They were asked the following 10 questions, each of them was to be answered by selecting out of 5 options, wherein option 1 was the most favorable and 5 the least favorable answer.

I) Did you have a good feeling in the mouth applying the gel?

II) How do you like the taste and the smell of the gel inside the mouth over the 7 days? Options for answering questions I) and II): 1=very comfortable, 2=comfortable, 3=neutral, 4=uncomfortable,5=very uncomfortable.

III) In case you experienced irritations after the tooth cleaning, did they go away after the applications of the gel?
1=completely, 2=almost completely, 3=partly, 4=rather a little, 5=rather not at all

IV) Would you like to use the gel again after the next tooth cleaning?
1=definitely, 2=almost definitely, 3=undecided, 4=rather not, 5=by no means.

V) Did the application of the gel lead to an improvement of the condition of the gingiva? 1=definitely, 2=probably,

3=undecided, 4=probably not, 5= definitely not.

VI) If you had tooth cleanings or dental clearances in the past, was the overall progress applying the gel...
1=very much better, 2=better, 3=the same, 4=worse, 5=very much worse than without applying the gel.

VII) How did you experience the further applications of the gel in the following days? 1=very comfortable, 2=comfortable, 3=neutral, 4=uncomfortable, 5=very uncomfortable.

VIII) Was the application of the gel with a cotton stick feasible? 1=definitely, 2=almost definitely, 3=undecided, 4=probably not, 5= definitely not.

IX) Were there any paresthesias or difficulties applying the gel?
1= completely impossible, 2= impossible, 3= undecided, 4= a little, 5= definitely.

X) Would you use the formulation as a gel/cleaning solution or as a spray solution also beyond the use after PTC?
1=guaranteed, 2= very likely, 3= undecided, 4= probably not, 5= definitely not.

The results of the survey are shown below. Further Table 2 also shows the % relative improvement of the Sulkus bleeding index, which was measured before PTC and after d7.
The % relative improvement of SBI is determined according to equation 1

$$\text{Equation 1:}\quad 100 - (SBI_{d7}/SBI_{beforePTC})*100$$

**Table 2: Average score of survey 7 days after PTC.**

| | Average score | | |
|---|---|---|---|
| **Question** | **Placebo** | **Reference 1** | **Formulation 1** |
| **I** | 3.2 | 3.1 | 2.3 |
| **II** | 2.8 | 3.3 | 2.6 |
| **III** | 3.5 | 3.3 | 2.0 |
| **IV** | 3.2 | 3 | 2.2 |
| **V** | 3.0 | 2.9 | 2.2 |
| **VI** | 3.2 | 3.1 | 2.2 |
| **VII** | 3.2 | 3.1 | 2.5 |
| **VIII** | 3.7 | 3.3 | 2.9 |
| **IX** | 2.8 | 3.2 | 2.5 |
| **X** | 3.3 | 3.3 | 2.0 |
| **% relative improvement sulkulus index** | -62 | 48 | 78 |

The study shows that formulation 1 is more effective to reduce irritations (question III) with an average score of 2.0 compared to the reference 1 (3.3) and the placebo (3.5). Further, the results show that, according to question V, the application of formulation 1 with an average score of 2.2 lead to a superior improvement of the condition of the gingiva compared to reference 1 with an average score of 2.9 and the placebo with an average score of 3.0.
As regards the relative improvement of the sulkulus index, formulation 1 provided a relative improvement of SBI of 78 % and thus a markedly better improvement compared to reference 1 and the placebo with a relative improvement of SBI of 48 % and 62 %, respectively.

**Example 5 individual case studies**

Formulation 1 has also been applied to patients in individual cases. The results were summarized in testimonials

for each individual case. Further, a particular interesting case of the PTC study was recorded.

**Example 5a Testimonial of case study 1**

Individual case, age 26, female, Herpes labialis, application at the lip, intra- und extraoral, application twice on the same day at 6pm and 8pm. The patient knew that she received an effective formulation and no placebo.

After applying the gel only twice within a period of 2 hours, the painful symptoms of Herpes Labialis disappeared. The Herpes was "gone" the next morning.

**Example 5b Testimonial of case study 2**

Individual case, age 11, female, recurring aphthae, intraoral application, multiple application within an afternoon and the following morning. The patient knew that she received an effective formulation and no placebo.

The patient experienced the first application as a little painful. The following application was not painful any more. At the end of the second day, the aphthae almost completely receded. The patient did not continue with further applications of the gel.

**Example 5c Testimonial of case study 4**

Individual case, age 75, female, irritation, weak inflammation of the gingiva, single application of gel. The patient knew that she was treated with an effective composition and not a placebo.

The patient considered the first application on the gingiva as comfortable. It was also considered to have a neutral smell. In a telephone interview on the following day, the patient reported a complete disappearance of the weakly painful irritations. The increased redness of the gingiva also receded.

**Example 6 Determination of average molecular weight of hyaluronic acid**

Size Exclusion Chromatography combined with multi angle light scattering (SEC-MALS) was performed using a Wyatt Heleos II 18 angle light scattering instrument coupled to a Wyatt Optilab rEX online refractive index detector. Samples for analysis were resolved on a Superdex S-200 10/300 analytical gel filtration column (GE Healthcare) running at 0.5 ml/min in in 50mM NaPi, 100mM NaCl, pH7.45 buffer before passing through the light scattering and refractive index detectors in a standard SEC MALS format.

Hyaluronic acid concentration was determined from the excess differential refractive index based on 0.155 ΔRI for 1 g/ml.

Samples were prepared for analysis by dissolving the Cube3® hyaluronic acid solid directly in running buffer at 2% w/v and subsequently diluted from this solution to load at 0.5 % w/v.

100 uL of sample were injected onto the SEC system and eluted.

Concentration and the observed scattered intensity were used to calculate absolute molecular mass using Wyatt's ASTRA software.

A sample of bovine serum albumin at 2mg/ml was run immediately before the hyaluronic acid measurements to check for column integrity and that inter detector delay times and system calibration were in order.

The Weight-average molar mass ($M_W$) was determined across the complete eluted peak area according to the Wyatt ASTRA software using the following equation.

$$M_w = \frac{\sum_i n_i M_i^2}{\sum_i n_i M_i} = \frac{\sum_i c_i M_i}{\sum_i c_i}$$

wherein $N_i$ is the number of molecules of weight $M_i$. and $c_i$ is equal to $n_i M_i$. The molecular weight of the Cube3® sample was in a range between about 200 kDa and about 3,500 kDa (Figure 1). The $M_W$ of the Cube3® sample was determined as 614.4 kDa.

The invention is not to be limited in scope by the specific embodiments disclosed in the examples that are

intended as illustrations of a few aspects of the invention and any embodiments that are functionally equivalent are within the scope of this invention. Indeed, various modifications of the invention in addition to those shown and described herein will become apparent to those skilled in the art and are intended to fall within the scope of the appended claims.

## Claims

**1.** A composition comprising

(i) hyaluronic acid or a salt thereof,
(ii) beta-glucan, and
(iii) optionally pullulan.

**2.** The composition of claim 1, wherein hyaluronic acid or salt thereof has a molecular weight ranging from about 50 kDa to about 3,500 kDa and/or
wherein the hyaluronic acid or salt thereof has a weight average molar mass from about 50 kDa to about 750 kDa.

**3.** The composition of claims 1 or 2, wherein the composition comprises from about 0.1 wt% to about 1 wt% hyaluronic acid or salt thereof.

**4.** The composition of any one of claims 1 to 3, wherein the hyaluronic acid or salt thereof is sodium hyaluronate.

**5.** The composition of claims 1 to 4, wherein

(i) beta-glucan has an average molecular weight from about 250 kDa to about 1250 kDa; and/or
(ii) the beta-glucan is chemically unmodified.

**6.** The composition of any one of claims 1 to 5, wherein the composition comprises

(i) from about 0.1 wt% to about 20 wt% beta-glucan; and/or
(ii) from about 0.1 wt% to about 10 wt% pullulan.

**7.** The composition of any one of claims 1 to 6, wherein the composition further comprises another naturally occurring film forming polymer.

**8.** The composition of claims 1 to 7, wherein the composition does not comprise (i) a chemically synthesized film forming agent and preferably not a chemically synthesized film forming agent selected from the group consisting of polyvinylpyrrolidone (PVP) acrylate copolymers, acrylate crosspolymers, acrylamide coplymers, acetyl triethyl citrate and polyvinylalcohol (PVA); and/or (ii) ethanol, polyethylene glycol, parabens, halogenated preservatives, synthetic dyes and/or synthetic sweeteners.

**9.** A composition of any one of claims 1 to 8 in the form of a gel, a paste, a spray solution, mouthwash, adhesive paste or powder.

**10.** A patch comprising the composition of any one of claims 1 to 9, wherein the patch further comprises a dissolvable polymer and wherein preferably

(i) the polymer dissolves inside the oral cavity and
wherein more preferably the polymer is dissolved or substantially dissolved within 15 minutes, within 30 minutes or within 60 minutes after topical application in the oral cavity; and/ or
(ii) the polymer is selected from the group consisting of cellulose derivatives, starch acetate and combinations thereof.

**11.** A composition of any one of claims 1 to 10, wherein the composition is (i) a cosmetic composition or (ii) a pharmaceutical composition.

**12.** Use of a composition of any one of claims 1 to 10 and 11 option (i) for oral hygiene.

**13.** Use of a composition of any one of claims 1 to 10 in the preparation of a pharmaceutical composition for topical application in the acute or prophylactic treatment of inflammatory affections, hypersensitivity, irritations or viral infections of the oral cavity;
and wherein preferably

(i) the inflammatory affection is selected from the group consisting of peridontitis, gingivitis, stomatitis and aphthous stomatitis, periimplantitis and mucositis; or
(ii) the acute or prophylactic treatment occurs after chemotherapy, radiotherapy or traumatizing events and more preferably
after professional tooth cleaning, dental surgery, chemotherapy or radiotherapy; or
(iii) the prophylactic or acute treatment is the treatment of Herpes infections, more preferably the treatment of Herpes labialis.

**14.** Composition of any one of claims 1 to 10 for use as a medicament and preferably for use in the acute or prophylactic treatment of inflammatory affections, hypersensitivity, irritations or viral infections of the oral cavity and wherein more preferably

(i) the inflammatory affection is selected from the group consisting of peridontitis, gingivitis, stomatitis, aphthous stomatitis, periimplantitis and mucositis or
(ii) the acute or prophylactic treatment occurs after chemotherapy, radiotherapy or traumatizing events; and more preferably
the acute or prophylactic treatment occurs after professional tooth cleaning, dental surgery, chemotherapy or radiotherapy; or
(iii) the prophylactic or acute treatment is the treatment of Herpes infections, preferably the treatment of Herpes labialis.

**15.** Use of hyaluronic acid with a molecular weight ranging from about 50 kDa to about 3,500 kDa or a salt thereof in the preparation of a pharmaceutical composition for topical application in the acute or prophylactic treatment of inflammatory affections, hypersensitivity, irritations or viral infections of the oral cavity and wherein the hyaluronic acid or salt thereof has a weight average molar mass from about 50 kDa to about 750 kDa.

Figure 1

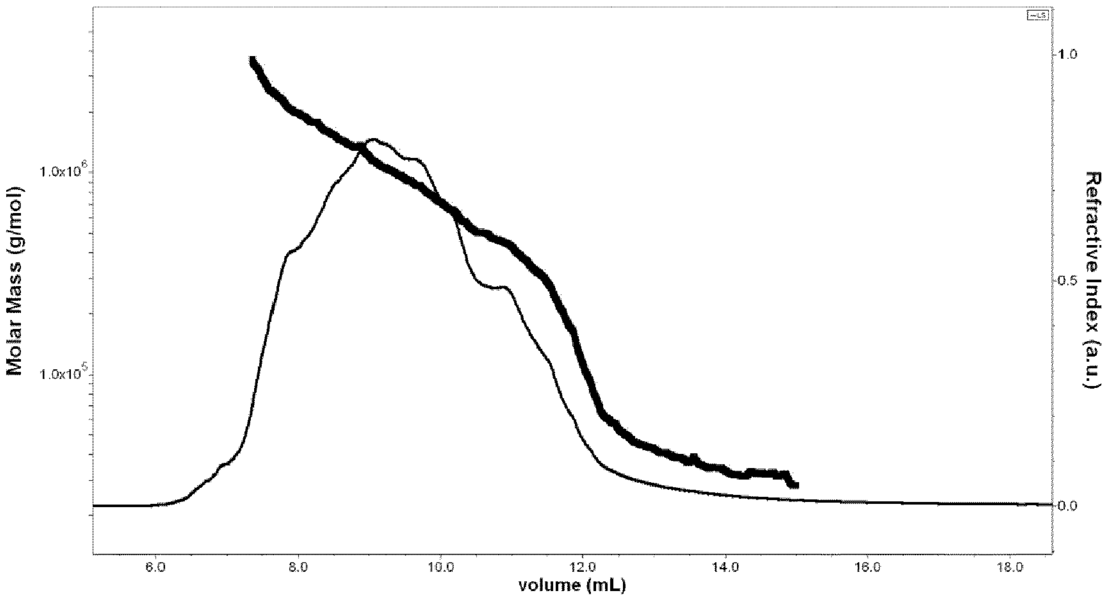

Molar Mass (g/mol)
1.0x10^6
1.0x10^5
Refractive Index (a.u.)
1.0
0.5
0.0
6.0
8.0
10.0
12.0
14.0
16.0
18.0
volume (mL)

Europäisches Patentamt
European Patent Office
Office européen des brevets

# EUROPEAN SEARCH REPORT

Application Number
EP 15 15 4971

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 0 444 492 A1 (SCHIENA RICERCHE [IT]; RICERFARMA SRL [IT]) 4 September 1991 (1991-09-04) * page 2, line 11 - line 4 * * page 2, line 45 - page 3, line 17 * * examples 2-4,6 * * claims 1-11 * ----- | 1-15 | INV. A61K9/00 A61Q11/00 A61K8/73 A61K31/716 A61K31/719 A61K31/728 A61K9/06 A61K8/04 ADD. A61K31/723 |
| X,D | WO 2005/000321 A1 (RICERFARMA SRL [IT]; MACCHI FRANCO [IT]) 6 January 2005 (2005-01-06) * page 1, line 4 - line 5 * * page 2, line 11 - page 3, line 7 * * example 1 * * claims 1-7 * ----- | 1-15 | |
| X | WO 2010/136872 A2 (PHARCOTERM S R L [IT]; PIZZONI ANGELO [IT]) 2 December 2010 (2010-12-02) * page 1, line 5 - line 11 * * page 4, line 16 - page 5, line 7 * * page 6, line 27 - line 14 * * page 8, line 10 - line 18 * ----- | 1,3-8, 11,13 | TECHNICAL FIELDS SEARCHED (IPC) A61K A61Q |
| X | US 5 972 906 A (ASCULAI SAMUEL SIMON [CA] ET AL) 26 October 1999 (1999-10-26) * column 2, line 25 - line 52 * * column 5, line 35 - line 49 * * claims 1-13 * ----- | 15 | |
| X | WO 2008/027904 A2 (REXADERM INC [US]; GOODHEART CLYDE RAYMOND [US]) 6 March 2008 (2008-03-06) * paragraph [0002] * * examples 1,2 * * tables 1,2 * * paragraph [0010] - paragraph [0015] * * claims * ----- -/-- | 1-15 | |

The present search report has been drawn up for all claims

| Place of search | Date of completion of the search | Examiner |
|---|---|---|
| The Hague | 15 July 2015 | Marchand, Petra |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 15 4971

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/069385 A1 (ROHTO PHARMA [JP]) 8 May 2014 (2014-05-08) * example 8 * ----- | 1,3,6-9, 11 | |
| A | WO 2012/087326 A1 (COLGATE PALMOLIVE CO [US]; PIMENTA PALOMA [US]; NESTA JASON [US]) 28 June 2012 (2012-06-28) * paragraph [0001] - paragraph [0006] * * claims 1,7,20 * ----- | 1-15 | |

TECHNICAL FIELDS SEARCHED (IPC)

The present search report has been drawn up for all claims

3

| Place of search | Date of completion of the search | Examiner |
|---|---|---|
| The Hague | 15 July 2015 | Marchand, Petra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 15 15 4971

This annex lists the patent family members relating to the patent documents cited in the above-mentioned European search report. The members are as contained in the European Patent Office EDP file on
The European Patent Office is in no way liable for these particulars which are merely given for the purpose of information.

15-07-2015

| Patent document cited in search report | | Publication date | Patent family member(s) | | | Publication date |
|---|---|---|---|---|---|---|
| EP 0444492 | A1 | 04-09-1991 | DE | 69116184 | D1 | 22-02-1996 |
| | | | DE | 69116184 | T2 | 05-06-1996 |
| | | | EP | 0444492 | A1 | 04-09-1991 |
| | | | ES | 2080844 | T3 | 16-02-1996 |
| | | | GR | 3019625 | T3 | 31-07-1996 |
| | | | IT | 1240316 | B | 07-12-1993 |
| WO 2005000321 | A1 | 06-01-2005 | AT | 422892 | T | 15-03-2009 |
| | | | BR | PI0411702 | A | 08-08-2006 |
| | | | CA | 2529441 | A1 | 06-01-2005 |
| | | | CN | 1812798 | A | 02-08-2006 |
| | | | CY | 1108992 | T1 | 02-07-2014 |
| | | | DK | 1638582 | T3 | 18-05-2009 |
| | | | EP | 1638582 | A1 | 29-03-2006 |
| | | | ES | 2322060 | T3 | 16-06-2009 |
| | | | JP | 4728955 | B2 | 20-07-2011 |
| | | | JP | 2009513492 | A | 02-04-2009 |
| | | | MX | PA05014184 | A | 09-03-2006 |
| | | | PT | 1638582 | E | 28-05-2009 |
| | | | SI | 1638582 | T1 | 31-10-2009 |
| | | | US | 2006147393 | A1 | 06-07-2006 |
| | | | WO | 2005000321 | A1 | 06-01-2005 |
| WO 2010136872 | A2 | 02-12-2010 | DK | 2435021 | T3 | 07-10-2013 |
| | | | EP | 2435021 | A2 | 04-04-2012 |
| | | | ES | 2428337 | T3 | 07-11-2013 |
| | | | PT | 2435021 | E | 30-09-2013 |
| | | | SM | T201300118 | B | 08-11-2013 |
| | | | WO | 2010136872 | A2 | 02-12-2010 |
| US 5972906 | A | 26-10-1999 | NONE | | | |
| WO 2008027904 | A2 | 06-03-2008 | BR | PI0716270 | A2 | 13-08-2013 |
| | | | EP | 2059206 | A2 | 20-05-2009 |
| | | | EP | 2489338 | A1 | 22-08-2012 |
| | | | US | 2009325861 | A1 | 31-12-2009 |
| | | | WO | 2008027904 | A2 | 06-03-2008 |
| WO 2014069385 | A1 | 08-05-2014 | NONE | | | |
| WO 2012087326 | A1 | 28-06-2012 | AR | 084582 | A1 | 29-05-2013 |
| | | | AU | 2010365779 | A1 | 09-05-2013 |
| | | | CA | 2822227 | A1 | 28-06-2012 |
| | | | CN | 103269673 | A | 28-08-2013 |
| | | | EP | 2654673 | A1 | 30-10-2013 |
| | | | JP | 2014501258 | A | 20-01-2014 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 15 15 4971

This annex lists the patent family members relating to the patent documents cited in the above-mentioned European search report. The members are as contained in the European Patent Office EDP file on
The European Patent Office is in no way liable for these particulars which are merely given for the purpose of information.

15-07-2015

| Patent document cited in search report | Publication date | Patent family member(s) | Publication date |
|---|---|---|---|
| | | RU 2013134360 A | 20-03-2015 |
| | | SG 190975 A1 | 31-07-2013 |
| | | TW 201238608 A | 01-10-2012 |
| | | US 2013272970 A1 | 17-10-2013 |
| | | WO 2012087326 A1 | 28-06-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0444492 A1
- WO 200500321 A1

### Non-patent literature cited in the description

- **MOMBELLI, A et al.** *Oral microbial Immunol.,* 1987, vol. 2, 145-151